# EUROPEAN PATENT SPECIFICATION

(11) **EP 1 407 264 B1**
(45) Date of publication and mention of the grant of the patent: **09.05.2007**
(21) Application number: 02750690.6
(22) Date of filing: 15.07.2002
(51) Int. Cl.: G01N 33/53, G01N 33/531, C12Q 1/66

(54) **CAGED COMPOUND CLEAVING PROCESS**
VERFAHREN ZUR SPALTUNG EINER IN EINEM KÄFIG GEFANGENEN SUBSTANZ
PROCEDE DE CLIVAGE DE COMPOSE "BLOQUES"

(30) Priority: 16.07.2001 CA 2353120
(43) Date of publication of application: 14.04.2004
(73) Proprietor: Cardiogenics Inc., Toronto, Ontario M9W 4Y9 (CA)
(72) Inventor: GAWAD, Yahia A., Mississauga, Ontario L4V 1H8 (CA)
(74) Representative: Perry, Robert Edward
(86) International application number: PCT/CA2002/001077
(87) International publication number: WO 2003/008969

(56) References cited:
- WO-A-00/79276
- WO-A-02/48393
- US-A- 5 451 683
- US-A- 5 942 407
- US-A- 6 136 268
- C.D. HODNELAND & M. MRKSICH: "Biomolecular surfaces that release ligands under electrochemical control" JOURNAL OF THE AMERICAN CHEMICAL SOCIETY, vol. 122, 2000, pages 4235-4236, XP002214886
- W-S. YEO, C.D. HODNELAND & M. MRKSICH: "Electroactive monolayer substrates that selectively release adherent cells" CHEMBIOCHEM, no. 7/8, 2001 - 27 July 2001 (2001-07-27), pages 590-593, XP002214887

## Description

### FIELD OF THE INVENTION

This invention relates to chemical complexes known as "caged compounds", and their use in initiating chemical and biochemical reactions. More specifically, it relates to procedures for cleaving caged compounds to release active chemical or biochemical components therefrom, and utilizing the released active entity in chemical or biochemical reactions such as biochemical assays.

### BACKGROUND OF THE INVENTION AND PRIOR ART

Several chemical groups have the convenient property that they can be removed or destroyed photochemically. Such photolabile chemical groups have been widely described and have been used in various applications. The photogeneration of an essential active chemical species in the course of a chemical reaction offers a milder method of cleavage than normally employed. As such, compounds containing photolabile chemical groups have been widely employed in organic and bioorganic reactions.

The ability to prepare photolabile compounds that modulate or block the activity of a critical reagent, for example adenosine triphosphate (ATP), and thus prevent its biochemical function allows localized delivery of reagent. This has been termed "chemical caging". Essentially, photolysis instantaneously releases the reactant *in situ* allowing the reaction to proceed.

Caged compounds are synthetic entities whose biological or biochemical activity is controlled by photocatalytic reaction. Caged compounds are most commonly designed by covalently coupling of a desired molecule (the "active moiety") with a suitable photoremovable protecting or "caging" group such that the activity of the active moiety is masked or caged. In one kind of chemical caging, on photoirradiation (photolysis) of appropriate wavelength, the photolabile bond is broken releasing an active moiety that could participate in a chemical or biochemical reaction such that it initiates the chemical or biochemical reaction in the immediate surrounding medium. In another kind of chemical caging, the photolabile bond is broken upon photoirradiation (photolysis) with the appropriate wavelength, releasing an active moiety that results in removal of one of the essential components of a chemical reaction. In other words, photocatalytic reactions employing caged compounds could either add or remove one essential component from a chemical reaction.

The term "caged " is utilized as an indication that a biologically or biochemically active species is trapped and masked inside a larger chemical "framework", and can be "released" upon illumination, thus uncaging the active content. The term "caged " has become popular because it is brief and pictorial, rather than being strictly accurate (see Adams et.al., Annu. Rev. Physiol. 55: 755-784, 1993.

One important application that utilizes photolabile chemical groups to cage and mask chemical moieties is the probing of biological systems. For example, photolysis of photolabile chemical groups of caged compounds and the consequent release of active chemical moieties involved in enzyme systems is one of the best techniques to examine the fast kinetics or spatial heterogeneity of biochemical responses in such systems. Illumination can be easily controlled in timing, location and amplitude. One can exert temporal and spatial control over the introduction of physiologically active compounds into complex systems, by introducing an inert photolabile compound such as a caged compound into the biological system and then activating it very rapidly with light. This provides a means of causing abrupt and localized changes in concentration of active species, in controlled amplitudes. This is particularly valuable when rapid mechanical mixing is impractical, for example on the surface of or inside a more or less intact cell, tissue, or protein crystal.

Caged compounds have been utilized to determine the structures of short-lived enzymatic intermediates, by using time-resolved Laue crystallography. In studying rapid enzymatic processes, numerous investigators have used caged compounds to mask an essential functional group, so that a chemical reaction may be initiated by a light pulse.

Many, but not all, of the photolabile protecting groups used in caging compounds comprise aromatic rings. To be useful in a biochemical system, a protecting group must satisfy several requirements. Caging of a compound with a photolabile protecting group should render the caged compound inert to the biochemical system used. The photolabile protecting group should release the active chemical moiety in high yield and at sufficient speed by photolysis at wavelengths not detrimental to the biochemical preparation. Further, photoproducts other than the active moiety should not interact with or interfere with the system. In nearly all useful caged biological molecules reported to date, simple covalent bond formation involving the active moiety masks some feature that is important for biological recognition. The photochemical cleavage of that covalent bond releases the active species (active moiety) or photoproducts having altered affinity, usually much reduced or much increased, to the active moiety.

The most frequently described caging compounds in the current literature are those based on the photoisomerism of 2-nitrobenzyl derivatives. The nitrobenzyl group is incorporated into the active molecule by linkage through a heteroatom, usually O, S or N. A wide range of other photolabile protecting groups is also commercially available.

In all of these photolabile protecting groups, the photoactivation process requires a light source. Any suitable conventional light source may be utilized to deliver a pulse of light energy to uncage photolabile compounds and release the active entity. Most commonly, lasers emitting energy in the ultraviolet (UV) or the infrared region are used. A brief, high flux emission of a light pulse results in the photoremoval of the photolabile protecting group. Also, UV flashlamps, which emit in the UV region of the spectrum or which their light output filtered to deliver UV radiation have been widely applied in the photochemistry of caged compounds.

The wavelength and energy of the optical source has been tailored to generate an appropriate pulse that breaks a particular photolabile bond of a caged compound. A non-exhaustive listing of photolabile chemical groups and the optimal wavelength for their removal may be found in the recently published (1998) *Methods in Enzymology* Volume 291 *"Caged* Compounds."

Also, the inclusion of the various photolabile chemical groups in a wide variety of chemical and biochemical entities of differing characteristics to produce caging compounds that mediate different functions in chemical or biochemical reactions is also to be found in the recent reference cited above.

Chemical caging with photolabile protecting groups has been employed in connection with amino acids, nucleic acids, enzyme substrates, catalysts of biochemical reactions and binding molecules whose affinities change upon irradiation. Among the common examples of active moieties employed in a caged compound form for use in biochemical systems are calcium ions, adenosine triphosphate (ATP), guanosine triphosphate (GTP), fluorescein and biotin.

A specific example of the use of caged compounds in biochemical processes is in bioaffinity binding assays such as immunoassays, nucleic acid binding assays and receptor binding assays. In such binding assays, the specific binding of affinity partners results in a modulation of a characteristic that may be easily measured. The modulated characteristics may be an enzymatic activity or a change in affinity that results in certain enzymatic activity. The modulated activity could also be measured through a change in certain characteristics prior to the binding reaction. Such a change could be the generation of light, modulation of colour absorbance or the generation of colour.

The affinity of binding of biotin to avidin or streptavidin is one of the strongest non-covalent bindings in chemistry. Both biotin and streptavidin have been successfully caged and utilized in photolytic reactions where their binding needs to be controlled in spatial or temporal terms. Also, both molecules have been successfully conjugated to various compounds in order to utilize the high affinity of these binding partners.

In United States patent 5,981,207 Burbaum et. al. disclose the utilization of caged enzyme substrates as probes in genetically-modified cells during *in vivo* cell-based reporter gene binding assays where the reporter enzyme activity and, by inference, the activator or suppressor activity of a compound under testing in a cell-based assay for drug discovery could be monitored. Also, in international patent application PCT/CA00/00718 Gawad discloses a process of monitoring *in vitro* binding assays by utilizing caged compounds. In both inventions, a chemiluminescent reaction is triggered by the photolytic release from a caged compound of an active moiety that participates in the binding assay. The resultant chemiluminescent signal is collected and monitored. In such photolabile reactions, the binding kinetics are closely controlled

A difficulty with such a process is the need for both a light source that triggers release of the active moiety needed for initiating the binding reaction signal from the caged compound and a light detection system to measure the emitted light output of the chemiluminescent reaction. Such light signals could interfere with each other, causing confusion between the triggering light signal (to cause uncaging of the caged compound) and the emitted light resulting from the chemiluminescent reaction. Moreover, the electronic detection system necessary to measure the light emissions, in the presence of a system providing light input, is complicated, cumbersome and expensive, if enough light emission is to be collected for meaningful measurements. Furthermore, the triggering light results in a decrease in the sensitivity of the binding assay due to a need for a light filtration process to separate the two different light signals.

Also known in the prior art are self-assembled monolayers having "caged" biotin moieties as pendant groups, which are released upon application of a "pulse" of reductive voltage. This reaction is used to trigger the affinity binding of streptavidin (via attached biotin) to the metal surface (C.D. Hodneland & M. Mrksich, (2000) J.Am.Chem.Soc. **122** 4235-4236).

U.S. patent 6,132,752 discloses a method for drug controlled release in which the drug is "caged" by ionic bond to a polypyrrol film having ionizable pendent moieties that release a proton upon application of a suitable voltage, thus breaking the ionic bond and releasing the drug.

U.S. patent 5,451,683 discloses a method for spatially-addressable immobilization of biomolecules on a surface which is based on a manufacture of an array of "caged" biotin molecules, which are protected with a group that is released upon irradiation with UV-VIS light or by oxido-reduction at an electrode surface.

### SUMMARY OF THE INVENTION

It has now been found that many if not all caged compounds can be cleaved to release the caged chemical moiety, in active condition, by being subjected to a pulse of high energy electric current. In accordance with the invention, instead of triggering release of the desired active moiety by photolysis, a totally different input is used, namely a high energy electric pulse, so that in a system where the active moiety is released from a caged compound and then this active moiety participates in a reaction for signal generation, particularly involving light generation, the input to release the active moiety cannot be confused with the output from the reaction. This leads to the adoption of simpler detection systems, and to more accurate measurements of light output.

Furthermore, in light-generating reactions where the method of triggering the release of the active moiety from the caged compound is other than a light pulse, an increase in the sensitivity of quantifying the reaction outcome can be achieved using less complicated machinery.

Thus according to a first aspect of the present invention, a process of releasing an active moiety from a caged compound in which said moiety is held in inactive form, comprises subjecting the caged compound to a pulse of DC high energy electric current.

According to a second aspect, in a process of conducting a binding assay for an analyte of interest, the signaling mechanism of the binding assay once activated results in the emission of light. According to this aspect, a process includes the steps of preparing, in an electrolyte medium, a mixture of a fluid containing or suspected of containing the said analyte, one or more specific binding partners for said analyte and other essential components of a light-generating signalling mechanism where one of said components is caged, releasing the active moiety from the caged compound, in active form, by subjecting the medium to a DC high energy electrical current pulse which results in uncaging of the active moiety from the caged compound and thereby initiating the light generating reaction, and measuring the emitted light signal of the signaling mechanism.

"Analyte" is a commonly used term of art, denoting a target compound whose presence and/or quantity is to be determined, in a test medium. The analyte is usually a necessary reactant in a reaction scheme. In assays for such an analyte, binding reactions are commonly used, based on bioaffinity or enzymatically catalyzed reactions. A specific binding partner known to have specific binding affinity for the analyte under test, for example an appropriately chosen antibody, natural hormone binding protein, lectin, enzyme, receptor, DNA, RNA or peptide nucleic acid (PNA), or artificial antibody or nucleic probe, is used, to form a complex with the analyte, and including a label to quantify the complex. The process of the invention, i.e. the subjection of a caged compound to high energy electric current to release the active ingredient from the caged compound, can be applied to provide to the reaction medium any of the active components required either to form the complex of the analyte and binding partner, or to activate the signal generating system.

### DESCRIPTION OF PREFERRED EMBODIMENTS

Caged compounds, which will undergo release of active moiety in response to subjection to high energy electric pulse for use in accordance with the invention, include substantially all of those previously reported in the literature having an organic protecting group and an active moiety that is photochemically releasable in active form. Preferred protective groups for caged compounds for use according to the invention include 2-nitrobenzyl; carboxy-2-nitrobenzyl; 2,2'-dinitrobenzhydryl; 1-(2-nitrophenyl)ethyl; 4,5-dimethoxy-2-nitrobenzyl; 1-(4,5-dimethoxy-2-nitrophenyl)ethyl; 5-carboxymethoxy-2-nitrobenzyl; ((5-carboxymethoxy-2-nitrobenzyl)oxy)carbonyl; (1-diazobenzyl)pyrene bromide; N-hydroxy-2-thiopyridone bromide; N-hydroxysuccinimidyl bromide; p-azidobenzoate, N-hydroxysuccinimidyl ester of p-azidobenzoylglycine bromide; N-hydroxysuccinimidyl bromide; (1-(2-nitro-4,5-dimethoxy)phenyl-diazoethane; 1-(2-nitro)phenyl-diazoethane; 1-(2-nitro-3,4,5,6-tetramethyl-diazoethane; desoxybenzoinyl; hydroxyphenacyl; 6-nitroveratryloxycarbonyl; 6-nitropiperonyloxy-carbonyl; alpha-dimethyl-dimethoxybenzyloxycarbonyl; 1-(4,5-dimethoxy-2-nitrophenyl)-1,2-diaminoethane-N,N,N,N-tetraacetic acid (DNMP); and 1-pyrenylmethyl. Some routine experimentation may be required, on the part of the skilled operator, to determine the best combinations of these protective groups with the preferred substrates for use in the present invention (ATP, GTP, Ca ions, etc), and to determine the optimum electric current pulse characteristics for their cleavage, as further discussed below.

The process of the invention utilizes a high energy electrical pulse to release an active moiety from the caged compound in active form through either breaking a chemical bond or through a change of the chemical affinity of the caging molecule before and after applying the electrical pulse. Preferably the electrical pulse is a direct current DC pulse, since use of an alternating AC current pulse entails detailed tuning of the frequency of the current to effect most efficient cleavage of the caged compound. No such problems are encountered with DC current, provided that the energy is sufficiently high to cleave the photolabile caging group, but not high enough to destroy one or more components of the chemical reaction. A minimum amount of direct electrical current is used to cleave the photolabile chemical moiety. Several parameters determine the level of energy delivered to the caging compound; the electrical current, the voltage, and the physical parameters of the system in which the reaction is conducted such as the shape of the electrodes to deliver the electrical current, the nature of the electrolyte, the material of the electrodes and the shape of the reaction vessel. All these factors determine the density of electrical current delivered to the photolabile chemical group. The amount of energy required to be furnished to effect the desired bond cleavage is related to the bond energy of the selected bond, but the relationship is not straightforward because of factors such as the nature of the electrolyte and the amount of the applied electrical energy which the electrolyte will absorb and hence will not reach the photolabile bonds.

The total energy supplied according to preferred embodiments of the invention is from about 0.01 m.joules to about 15 joules. The energy supplied is dependent upon the time for which the current is delivered, as well as the strength of the current. For example, if the DC current supplied is of high voltage (300 volts and above), the duration of the pulse required to cleave the caged compound can be as short as one microsecond. When a lower voltage is used, e.g. 70 volts, a pulse duration of one microsecond will only release a portion of the active moiety from the caged compound, and repeated pulses of such duration are required to release all of the caged compound. There are occasions when the release of only a portion of the caged active moiety is desirable in order to control various aspects of the chemical reaction. Longer pulses do not appear to cause significant problems. A low voltage (4.8V, for example) for a longer pulse (3.3 seconds) has been satisfactorily used in practice. Use of such low voltages and longer times minimizes the loss of energy, which might otherwise heat up the liquid medium. Some routine experimentation with the chosen system, to determine the optimum electrical input, may be desirable, but such experimentation is well within the skill of the art.

In practice, a preferred method of conducting the process of the present invention is to utilize a reaction cell containing two spaced-apart current-delivering electrodes between which the current can be passed through an electrolyte. The cell is filled to an appropriate extent with an electrolyte medium containing all the needed reaction components including the caged compound. Upon delivery of the DC current, the photolabile chemical group of the caged compound releases the active moiety needed to initiate the desired chemical reaction. In light-generating chemical reactions in which one of the reaction components is a caged compound, a light receiving detection system is provided, to receive and quantify light emissions from the reaction solution.

Appropriate electrical circuitry to provide pulsed DC electric current, of predetermined voltage and duration, and hence energy level, is connected to the electrodes, and activated to cause cleavage of the caged compound. Incident light is not used to cause cleavage of the caged compound, and so no special measures such as light-proof shutters or light filtering or beam-splitting devices, are needed to collect light emitted in the light-producing chemical reaction.

The process of the invention shows utility not only in causing luminescent emissions from a chemical reaction in binding assays as described above, but also in other areas where a detectable change due to uncaging of a caged compound and release of an active moiety which is essential for the progression of a chemical reaction is observed.

Further, the process of the invention provides controlled spatial or temporal delivery of one component of a reaction system, which obviates designing complicated mechanical delivery mechanisms of such a component. The variety of chemical reactions, which might benefit from the process of the invention include many binding assays. For example, determination of bacterial contaminants in food, where antibodies to the bacteria can be bound to a solid substrate and bind selectively to a chosen enzyme which subsequently reacts with the released active component from the caged compound and a detectable change in the enzyme activity is measured.

The invention is further described, for illustrative purposes only, in the following specific experimental examples.

### Specific Embodiments of the Method of the Invention.

The method of the invention is the trigger of a chemical reaction upon delivery of an electric pulse to the reaction medium. As a prerequisite to employ the method of the invention for the initiation the chemical reaction is the presence of caged chemical compound, which is either inactive when in the caged condition or carry a trigger compound rendering the trigger compound inaccessible to activate the reaction. Upon employing the method of the invention of activation of the reaction by an electrical current pulse, the chemical reaction is initiated by releasing an active compound from the caged inactive precursor. Caged compounds offer a chemical entrapment method for delivery of reagents, which is superior to other delivery methods such as physical entrapment of chemical reagents (e.g. in liposomes) or delivery by mechanical means.

Certain chemical reactions could benefit from the method of the invention of applying an electric current to release active compounds from caged compounds needed for the chemical reaction to proceed by either speeding up the reaction, control of reaction dynamics or by simplifying the machinery involved in timed-sensitive addition of reagents. A general scheme of a simple chemical reaction is a follow:

**A+B ⇒ C**

Whether C is the final reaction product and can be measured, or whether C is linked to another chemical reaction for generating a measurable signal that quantifies this reaction, the method of the invention can be employed. All the reaction components are essential for a measurable outcome and any or all of the components can be caged with photolabile bond and can be released by a high-energy direct current electrical pulse in accordance with the invention, supplied by appropriate electrical circuitry.

A requisite of the reaction to benefit from the method of the invention is that mixing all of the reactants together, with one or more of the reagents being caged, facilitates no progression of the reaction. Release of the caged component as an active moiety that is needed for the chemical reaction to proceed by means of the method of the invention results in the production of a measurable product. Depending on the specifics of the electrical pulse supplied in the process of the invention, the rate of the reaction can be controlled.

Examples of various chemical reactions that result in a measurable outcome and can benefit from the method of the invention of releasing caged compounds, include light-producing chemical reactions e.g. those involving enzymes and visible outcome chemical reactions. Specific examples of the various kinds of chemical reactions that can benefit from the method of the invention are as follows:

### Example 1: Light producing chemiluminescent reactions.

Numerous chemiluminescent reactions are known and utilized in research and clinical laboratories. Caging of one or more compound needed to initiate a chemiluminescent reaction and the release of the needed compound by the method of the invention offers control over the speed and amplitude of light generation and also over the timing of release of an active chemical compound.

For example, in the chemiluminescent reactions, which use photoproteins, initiation of the reaction depends on the addition of Ca to the reaction mixture. A photoprotein chemiluminescent reaction can be illustrated by the following equation:

**Photoprotien + Luciferin + Ca + Oxygen ⇒ Light**

Addition of Ca to the charged photoprotein (photoprotein already bound to luciferin) results in an instantaneous chemical reaction and the generation of light. Substituting Ca by a caged Ca compound and triggering the release of the caged Ca by electrical pulse according to the invention will initiate the chemiluminescent reaction and the generation of light.

Depending on the electrical pulse characteristics, the light generation from the chemiluminescent reaction can be a single flash of light, or several flashes, or a steady light emission depending on the amounts of released Ca as determined by the characteristics of the electrical pulse used in the method of the invention.

To confirm that the electrical current pulse altered the photolabile caging compound and caused release of Ca to trigger photoprotein chemiluminescence, one of the reaction components was omitted from the reaction chamber. When the photolabile Ca caging compound was omitted from the reaction, no chemiluminescent light generation occurred. When the photoprotein is omitted, no chemiluminescent light emission occurs. However, adding the photoprotein after the electrical pulse generates chemiluminescent light.

Further, to confirm that this process is specific to the alteration of the caging compound and release of Ca to trigger the reaction, a non-photolabile Ca chelating agent (EDTA), which has a higher affinity for Ca than the photoprotein, was added to the reaction cell that contains all other reaction components, including the Ca-caging compound. Under such condition, no light generation occurred as the released Ca is chelated by the non-photolabile Ca chelating agent.

Further, to confirm the specificity of unloading of the photolabile trigger compound by this form of energy, another from of energy was utilized in an attempt to trigger the same chemiluminescent reaction in the presence of a Ca-caging compound. Triggering an electromagnetic field of an electromagnet through the same electric circuitry resulted in no generation of light.

Examples ofphotoproteins are aequorin, obelin, mnemiopsin, berovin, phosalin, luciferase of ostracods and cypiridina.

Another example chemiluminescent reaction where the method of the invention is useful is chemiluminescent reactions that employ the luciferases. Firefly luciferase-mediated chemical reaction, which generate light could be exemplified as follows:

**Luciferase + Luciferin + ATP + Mg + Oxygen ⇒ Light**

During luciferase-mediated chemiluminescent light-generating reactions, one or more essential components of the reaction can be in a caged form. With all the reaction components present, utilizing the method of the invention for uncaging of a caged molecule induces the generation of light from the luciferase chemiluminescent reaction. Upon triggering the reaction by the method of the invention of electrical current pulse to trigger the reaction, a controlled release of the caged component or components occurs and the released active moieties will trigger the reaction and result in light generation. Modulating the current electrical pulse to release one or more of the caged components in a controlled way would result in light emission that can be monitored.

Normally, a luciferase-mediated chemiluminescent reaction results in the release of a burst of light which is difficult to measure and monitor as it lasts only a second at most. Several prior art patents have disclosed methods to alter the light output by adding one or more cofactors to the reaction. By utilizing the method of the invention, controlling the release of the caged compound controls the amount of released light and also simplifies the machinery needed to monitor the light emission. Several components of the chemiluminescent system of the luciferase enzyme are already commercially available in a caged form, such as the luciferase enzyme itself, luciferin, and ATP. Also, caged chelating agents can be utilized to cage Mg. Most of these are available from Molecular Probes (Eugene).

### Example 2: Non-chemiluminescent light emitting reactions.

In fluorescent binding reactions, the sensitivity of binding assays is limited by the fluorescence of the medium where the reaction is carried out as well as the container. The high non-specific background signal limits the lower limit of detection of fluorescent assays. It has been suggested that bleaching the non-specific fluorescence of the reaction medium before stimulating the fluorescence of the specific signal would result in a lower background, thus lowering the lower limit of detection. Several caged fluorescent compounds have been developed for this purpose. Irradiating the medium where a caged fluorescent compound is present would result in bleaching of fluorescence of the medium and at the same time maintain the caged fluorescent compound without exhaustion. Uncaging fluorescent compounds using the method of the invention will then simplify the machinery needed to gather the emitted signal, since otherwise the caged compounds need to be irradiated with UV light and most of the stimulation spectra of the modem fluorescent compounds are in the visible range. Therefore, utilizing the method of the invention would simplify the optical components of various detection systems.

### Example 3: Colour Enzymatic Reactions.

The method of the invention of unloading or uncaging caged compounds to release the active moiety through he utilization of a high energy electrical pulse can be employed in binding assays with enzyme-mediated color changes. Numerous binding reactions and binding assays utilize enzymes to result into a measurable colour changes that indicate the quantity of the chemical entity under study. In most of these reactions, an enzyme-catalyzed process results in the conversion of a substrate from one color to another. The amount of color change then indicates the quantity of the chemical entity. During such reactions, adding one or more components to the system trigger initiation of the reaction. Commonly, this step is carried out mechanically. Replacing the mechanical step with the method of the invention would result in simplifying the measuring machinery.

The method of the invention can also be utilized in binding assays where a caged compound can be released by an electrical pulse and when the measured property is not an electrical signal. In almost any biological system where the measured output is not an electrical signal, the method of the invention can be utilized to trigger the cleavage of the photolabile bonds of various caging compounds. For example, the method of the invention could be employed in cell mediated binding assays where a caged compound can be released by an electrical pulse. The list of applications in this area is very broad.

### Experimental Results

The method of the invention was demonstrated by carrying out several chemiluminescent reaction experiments.

### Experiment 1

In one experiment, in a total reaction volume of 10 µL, all the components of a photoprotein chemiluminescence reaction were added in suitable electrical cell (Aequorin, native or recombinant, and recombinant Obelin were utilized in amounts varying from 0.5 - 6 micrograms). The reaction cell also contained Ca-caging compound loaded with Ca to such an extent that the level of free Ca does not trigger light emission. Specifically, the Ca-caging compound was DNMP saturated to an extent of 50% - 77% with Ca. Two spaced metal electrodes were connected to a suitable circuitry to deliver a DC electrical pulse. The electrical pulse characteristics were changed and the light emission from reaction was monitored. Various metals were used in the different experiments, namely silver, aluminum and steel, and various different shapes of electrode, cylindrical, U-shaped, etc were used. A variety of different buffered electrolyte solutions (to decrease changes in pH due to the released compounds), were employed. These included MOPS buffer with 80mM KCl (pH7.4 and 7.2), serum and plasma. All experiments were operated successfully. The following table summarizes the results.

| Pulse voltage. (V) | Pulse duration. (S) | Peak current. (mA) | Number of pulses/Light flashes |
|---|---|---|---|
| 320 | 0.012 | 70 | 1 |
| 150 | 0.52 | 47 | 1 |
| 100 | 1.1 | 54 | 1 |
| 70 | 1.15 | 22.8 | >5 |
| 63 | 1.1 | 19.5 | 15 |
| 60 | 0.52 | 29.6 | 11 |
| 50 | 2.4 | 17.6 | >5 |
| 46 | 2.6 | 15.9 | >8 |
| 24 | 2.4 | 40.9 | >6 |
| 12 | 2.2 | 30.1 | 3 |
| 5 | 3.3 | 5.7 | 3 |

In each of the previous experiments, altering the shape and/or material of the electrodes to deliver an electrical pulse of a certain voltage caused the characteristics of the light emission profile to change, but in all cases the experiment proceeded successfully. Replacing the electrolyte medium with pure water resulted in unsuccessful experiments.

### Experiment 2

To confirm that the electrical pulse induces unloading of the photolabile Ca-caging compound and release of Ca, which triggers light emission of phortoproteins, one of the reaction components was omitted from the reaction. When the photolabile Ca-caging compound was omitted, no chemiluminescent light generation occurred. When the photoprotein (Aequorin or Obelin) was omitted from the reaction, no chemiluminescent light emission also occurred. However, adding the photoprotein after the electrical pulse of the caged Ca initiated chemiluminescent light generation. In these experiments, the total electrical energy used is 5.94J.

### Experiment 3

Further, to confirm that this process is specific to unloading of the photolabile Ca-caging compound and release of Ca to trigger the chemiluminescent reaction, a non-photolabile Ca-chelating agent (EDTA), which has a higher affinity to Ca than photoproteins, was added to the reaction cell that contains all the other reaction components. Under such condition, no light generation occurred as the released Ca is chelated by the non-photolabile Ca chelating agent. In this experiments, the electrical energy used was 5.94J.

### Experiment 4

To confirm the specificity of the destruction of the photolabile compound to this form of energy, a magnetic field was generated from pulsed electromagnet through the same electric circuitry used to generate chemiluminescence. In case of the electromagnetic field pulse, no light generation occurred.

### Experiment 5

The generation of light from a chemiluminescent reaction that employ the method of the invention depends on the characteristics of the electrical pulse with regard to its duration as well as amplitude. In order to demonstrate this dependency, a different electrical circuitry was employed. An electrical circuit that relies on the fast discharge of electrical capacitor was employed to generate light of a chemiluminescent reaction by the method of the invention. Capacitors with voltage values between 100-330 Volts and capacitances of between 1-220 µF were utilized. The shape of the electrical pulse of the capacitor discharge determines the characteristics and frequency of the light flash or emission. Further, another kind of electrical circuitry was employed to demonstrate the method of the invention. A DC power supply source with an output voltage between 3 and 150V and a switching circuit to control the duration of the pulse at which a certain voltage was applied was employed to trigger light generation of photoproteins Aequorin and Obelin chemiluminescence. Applying different electric pulses with characteristics as listed in the previous table resulted in triggering of light emission. Furthermore, applying a direct current electrical pulse below the required characteristic generated no light from the same reaction in the same electrical cell. Several pulses had to be applied before light emission.

### Experiment 6:

The method of the invention was also employed for triggering light emission from a chemiluminescence reaction that employs other caged reagents. Light emission of the chemiluminescence reaction of the luciferase was utilized with various caged compounds that are needed to trigger the reaction. A typical firefly luciferase chemiluminescent reaction needs all the following essential components; Luciferase enzyme, Luciferin, Magnesium and ATP, in the presence of oxygen to generate light according to the following reaction:

Luciferase + D-Luciferin + ATP + Mg⁺⁺ → Oxidized Luciferin + pyrophosphate + CO2 + Light.

The utility of method of the invention was demonstrated by carrying out the previous luciferase chemiluminescent reactions with one of the components of the reaction being caged. Upon delivering the needed electrical pulse, the caged compound is uncaged causing an instantaneous release of an active compound and the triggering of light generation.

A caged ATP was utilized to demonstrate the method of the invention of controlling the trigger of light generation by a pulse of electric current. Caged ATP is not an active substrate of the luciferase chemiluminescence, however, functional ATP, which acts as a substrate for the luciferase reaction could be delivered by the method of the invention.

In a total reaction volume of 12 µL, the following are mixed in a suitable electric reaction cell: Luciferase/D-Luciferin solution mix (6µL), 5mM Mg Citrate in PBS (3µL), caged ATP solution (3µL). The electrodes were connected to a power supply circuitry and an electric pulse was triggered to uncage ATP and initiate light generation. Various voltages and pulse durations were utilized. Under these experimental conditions, employing the method of the invention resulted in the generation of light from the luciferase chemiluminescence reaction.

Also, in order to demonstrate that the method of the invention can be employed with various caging compound, as long as the electrical pulse shape is altered, another caged compound employed in luciferase chemiluminescence reaction was utilized. In this experiment, caged D-Luciferin was utilized to control the reaction kinetics. Functional D-Luciferin was delivered to the reaction from caged D-Luciferin upon exposure of the reaction components to an electric pulse.

In a total reaction volume of 25 µL, the following components were added as solutions to a suitable electric cell: Luciferase solution (10 µL), 5mM Mg Citrate in PBS (5µL), 1mM ATP solution (5µL) and Caged D-Luciferin solution (5µL). A suitable electrical circuitry was connected to the cell and an electric pulse was delivered. Upon delivery of the electric pulse, the caged compound released the active component needed to trigger light generation of the luciferase chemiluminescence reaction. The experimental results of both the luciferase chemiluminescence reactions with the two caged compounds; caged ATP and caged Luciferin are summarized as follows:

| Pulse voltage (V) | Pulse duration (S) | Peak current. (mA) | Number of pulses/ Light flashes |
|---|---|---|---|
| 350 | 0.184 | 40.9 | 1 |
| 100 | 0.300 | 32.2 | 1 |
| 50 | 1.1 | 29.3 | 1 |

In these experiments of the luciferase chemiluminescence, an electrical circuit that relies on discharge of an electrical capacitor at various voltages was employed. Capacitors of a voltage value between 50-350 volts and capacitances of between 10-150 uF were utilized to alter the shape of the electric pulse. It was observed that a time lag of about 0.5 second is needed before light emission start from the reaction. It was assumed that light generation start immediately, however the amounts of released active trigger compound from the caged compound need to accumulate before substantial enzyme-activated reaction and therefore light emission could be observed.

### Reagents:

- DM-EDTA(1-(4,5-Dimethoxy-2nitrophenyl)-1,2-diaminoethane-N,-N,-N,-Ntetra acetic acid), Molecular Probes, Eugene, OR, USA (catalog # D-6814).
- Recombinant Aequorin, Aqualite, Molecular Probes, Eugene, OR, USA (Catalog # A-6785).
- Native Aequorin, Friday Harbor Photoproteins, Friday Harbor, WA, USA.
- Recombinant Obelin, curtsey of Dr. Eugene Vysotski, Dept. of Biochemistry, University of Georgia, Georgia, USA.
- A lyophilized mix of Luciferase/D-Luciferin is dissolved in Tricine reconstitution buffer [50 mM N-Tris(hydroxymethyl) methylglycine, adjusted with NaOH to pH 7.8, Lot 1418], both supplied by Kikkoman as assay kit (CheckLite HS Plus, Catalog # 60342).
- Luciferase enzyme dissolved in Tricine buffer pH 7.8 [(50 mM N-Tris (hydroxymethyl) methylglycine] adjusted with NaOH, supplied by Kikkoman Catalog LUC T).
- 5 mM Mg Citrate solution in Phosphate buffered saline (PBS, pH 7.4).
- Caged ATP in methanol, 5mg in 300 µL (Molecular Probes, Eugene, OR, USA Catalog # A-1049).
- Caged D-Luciferin 5mg dissolved in 300 µL of Dimethylsulfoxide (DMSO) (Molecular Probes, Eugene, OR, USA, Catalog # L-7085)
- 100mM ATP solution, pH 7.5 (Amersham Pharmacia, Catalog # 272056).

## Claims

1. A process of releasing an active moiety from a caged compound in which said moiety is held in inactive form, which comprises subjecting the caged compound to a pulse of DC high energy electric current.

2. The process of claim 1wherein the caged compound is dissolved or suspended in an electrolyte medium also containing at least one reagent which is chemically reactive towards said active moiety, at the time of subjection of the caged compound to said pulse of DC high energy electric current.

3. The process of claim 2 wherein said active chemical moiety and said at least one reagent are chosen so that their chemical reaction results in a measurable signal.

4. The process of claim 3 wherein the measurable signal is the emission of light.

5. The process of any preceding claim wherein the DC electric current pulse supplies from about 0.01 m-joules to about 15 joules total energy.

6. The process of any preceding claim wherein the caged compound has aromatic ring-containing protective groups.

7. The process of claim 6 wherein the caged compound comprises a 2-nitro benzyl group linked to the active moiety through a hetero atom.

8. The process of any preceding claim wherein the active moiety is a compound needed to trigger a chemical reaction with a measurable outcome.

9. The process of claim 8 wherein the active moiety is a calcium ion, ATP , GTP, fluorescein, biotin or streptavidin.

10. A process of conducting a binding assay for an analyte of interest where the signalling mechanism of the binding assay once activated results in the emission of light, said process including the steps of preparing, in an electrolyte medium, a mixture of a fluid containing or suspected of containing the said analyte, one or more specific binding partners for said analyte and other essential components of a light-generating signalling mechanism wherein one or more of said components is caged, releasing the active moiety from the caged compound, in active form, by subjecting the medium to a high energy DC electrical current pulse which results in uncaging of the active moiety from the caged compound and thereby initiating the light generating reaction, and measuring the emitted light signal of the signalling mechanism

11. The process of claim 10, wherein the DC electric current pulse supplies from about 0.01 m-joules to about 15 joules total energy.

12. The process of claim 10, wherein the signalling mechanism is light generation by a photoprotein chemiluminescent reaction.

13. The process of claim 10, wherein the signalling mechanism is light generation by a luciferase chemiluminescent reaction.

14. The process of any of claims 10 or 11, wherein the mixture comprises the analyte linked to or conjugated with a photoprotein reactable with luciferin, a caged compound catalyst, and luciferin, the photoprotein-luciferin reaction resulting in chemiluminescence.

15. The process of claim 14, wherein the catalyst is calcium.

16. The process of claim 14, wherein the photoprotein is aequorin, obelin, mnemiopsin, berovin, phosalin, luciferase of ostracods or cypridina.

17. The process of claim 16, wherein the analyte is linked to or conjugated with luciferase enzyme for generating a measurable outcome.

18. The process of claim 10, wherein the caged compound is catalytic caged ATP.

19. The process of claim 10, wherein the caged compound is caged luciferin.

## Patentansprüche

1. Verfahren zur Freisetzung einer aktiven Komponente aus einer Einschlussverbindung (Caged Compound), in der die Komponente in inaktiver Form gehalten wird, welches das Unterwerfen der Caged Compound einem hochenergetischen elektrischen Gleichstromimpuls umfasst.

2. Verfahren nach Anspruch 1, bei dem die Caged Compound in einem Elektrolytmedium gelöst oder suspendiert ist, das auch mindestens ein Reagenz enthält, das hinsichtlich der aktiven Komponente dann chemisch reaktiv ist, wenn die Caged Compound dem hochenergetischen elektrischen Gleichstromimpuls unterworfen wird.

3. Verfahren nach Anspruch 2, bei dem die aktive chemische Komponente und das mindestens eine Reagenz so ausgewählt werden, dass ihre chemische Reaktion zu einem messbaren Signal führt.

4. Verfahren nach Anspruch 3, bei dem das messbare Signal die Emission von Licht ist.

5. Verfahren nach irgendeinem vorhergehenden Anspruch, bei dem der elektrische Gleichstromimpuls etwa 0,01 mJoule bis etwa 15 Joule Gesamtenergie zuführt.

6. Verfahren nach irgendeinem vorhergehenden Anspruch, bei dem die Caged Compound einen aromatischen Ring enthaltende Schutzgruppen aufweist.

7. Verfahren nach Anspruch 6, bei dem die Caged Compound eine 2-Nitrobenzyl-Gruppe umfasst, die über ein Heteroatom mit der aktiven Komponente verknüpft ist.

8. Verfahren nach irgendeinem vorhergehenden Anspruch, bei dem die aktive Komponente eine Verbindung ist, die notwendig ist, um eine chemische Reaktion mit einem messbaren Ergebnis auszulösen.

9. Verfahren nach Anspruch 8, bei dem die aktive Komponente ein Calciumion, ATP, GTP, Fluorescein, Biotin oder Streptavidin ist.

10. Verfahren zur Durchführung eines Bindungsassays für einen interessierenden Analyten, bei dem der einmal aktivierte Signalmechanismus des Bindungsassays zu Lichtemission führt, wobei das Verfahren die Schritte des Herstellens einer Mischung von einem Fluid enthaltend oder im Verdacht stehend zu enthalten den Analyten, einen oder mehrere spezifische Bindungspartner für den Analyten und andere wesentliche Komponenten für einen Licht erzeugenden Signalmechanismus in einem Elektrolytmedium, wobei eine oder mehrere der Komponenten eingeschlossen sind, der Freisetzung der aktiven Komponente aus der Caged Compound in aktiver Form, indem das Medium einem hochenergetischen elektrischen Gleichstromimpuls ausgesetzt wird, der zur Befreiung der aktiven Komponente aus der Caged Compound und **dadurch** zur Initiierung der Lichterzeugungsreaktion führt, und des Messens des emittierten Lichtsignals des Signalmechanismus beinhaltet.

11. Verfahren nach Anspruch 10, bei dem der elektrische Gleichstromimpuls etwa 0,01 mJoule bis etwa 15 Joule Gesamtenergie zuführt.

12. Verfahren nach Anspruch 10, bei dem der Signalmechanismus eine Lichterzeugung durch eine Photoprotein-Chemilumineszenzreaktion ist.

13. Verfahren nach Anspruch 10, bei dem der Signalmechanismus eine Lichterzeugung durch eine Luciferase-Chemilumineszenzreaktion ist.

14. Verfahren nach irgendeinem der Ansprüche 10 oder 11, bei dem die Mischung den Analyten, der mit einem mit Luciferin reaktionsfähigen Photoprotein verknüpft oder konjugiert ist, einen Caged Compound-Katalysator und Luciferin umfasst, wobei die Photoprotein-Luciferin-Reaktion zur Chemilumineszenz führt.

15. Verfahren nach Anspruch 14, bei dem der Katalysator Calcium ist.

16. Verfahren nach Anspruch 14, bei dem das Photoprotein Aequorin, Obelin, Mnemiopsin, Berovin, Phosalin, Luciferase von Muschelkrebsen oder Cypridina ist.

17. Verfahren nach Anspruch 16, bei dem der Analyt zur Erzeugung eines messbaren Ergebnisses mit Luciferase-Enzym verknüpft oder konjugiert ist.

18. Verfahren nach Anspruch 10, bei dem die Caged Compound katalytisches caged ATP ist.

19. Verfahren nach Anspruch 10, bei dem die Caged Compound caged Luciferin ist.

## Revendications

1. Procédé de libération d'une fraction active d'un composé encagé, dans lequel ladite fraction est retenue sous forme inactive, lequel procédé comprend l'étape qui consiste à appliquer au composé encagé une impulsion de courant électrique continu à haute énergie.

2. Procédé selon la revendication 1, dans lequel le composé encagé est dissous ou en suspension dans un milieu électrolytique qui contient aussi au moins un réactif qui réagit chimiquement avec ladite fraction active, à l'instant où le composé encagé subit ladite impulsion de courant électrique continu à haute énergie.

3. Procédé selon la revendication 2, dans lequel ladite fraction chimiquement active et ledit au moins un réactif sont choisis de manière à ce que la réaction chimique délivre un signal mesurable.

4. Procédé selon la revendication 3, dans lequel le signal mesurable est l'émission de lumière.

5. Procédé selon l'une des quelconques revendications précédentes, dans lequel l'impulsion de courant électrique continu délivre une énergie totale comprise entre environ 0,01 millijoule et environ 15 joules.

6. Procédé selon l'une des quelconques revendications précédentes, dans lequel le composé encagé présente des groupes protecteurs qui contiennent un cycle aromatique.

7. Procédé selon la revendication 6, dans lequel le composé encagé comprend un groupe 2-nitrobenzyle lié à la fraction active par un hétéroatome.

8. Procédé selon l'une quelconque revendication précédente, dans lequel la fraction active est un composé nécessaire pour déclencher une réaction chimique dont le résultat est mesurable.

9. Procédé selon la revendication 8, dans lequel la fraction active est un ion de calcium, de l'ATP, du GTP, de la fluorescéine, de la biotine ou de la streptavidine.

10. Procédé qui consiste à réaliser une détermination de liaison d'un analyte auquel on s'intéresse et dans lequel le mécanisme de signalisation de la détermination de liaison entraîne l'émission de lumière une fois qu'il est activé, ledit procédé comprenant les étapes qui consistent à préparer un mélange d'un fluide qui contient ou dont on suspecte qu'il contient ledit analyte dans un fluide électrolytique, un ou plusieurs partenaires de liaison spécifiques pour ledit analyte et d'autres composés essentiels d'un mécanisme de signalisation par émission de lumière, un ou plusieurs desdits composants étant encagés, à libérer la fraction active du composé encagé sous une forme active, à appliquer au fluide une impulsion de courant électrique continu à haute énergie, ce qui entraîne la libération de la fraction active du composé encagé et ainsi amorce la réaction de génération de lumière, et à mesurer le signal de lumière émis par le mécanisme de signalisation.

11. Procédé selon la revendication 10, dans lequel l'énergie totale de l'impulsion de courant électrique continu est comprise entre environ 0,01 millijoule et environ 15 joules.

12. Procédé selon la revendication 10, dans lequel le mécanisme de signalisation est une émission de lumière par une réaction de chimioluminescence d'une photoprotéine.

13. Procédé selon la revendication 10, dans lequel le mécanisme de signalisation est une émission de lumière par une réaction de chimioluminescence de luciférase.

14. Procédé selon l'une quelconque des revendications 10 ou 11, dans lequel le mélange comprend l'analyte lié ou conjugué à la photoprotéine qui réagit avec la luciférine, un catalyseur de composé encagé et de la luciférine, la réaction photoprotéine-luciférine entraînant une chimioluminescence.

15. Procédé selon la revendication 14, dans lequel le catalyseur est le calcium.

16. Procédé selon la revendication 14, dans lequel la photoprotéine est l'aéquorine, l'obéline, la mnémiopsine, la bérovine, la phosaline, la luciférase d'ostracodes ou la cypridine.

17. Procédé selon la revendication 16, dans lequel l'analyte est lié ou conjugué à l'enzyme luciférase afin de générer un résultat mesurable.

18. Procédé selon la revendication 10, dans lequel le composé encagé est un ATP catalytique encagé.

19. Procédé selon la revendication 10, dans lequel le composé encagé est une luciférine encagée.
